(19) Europäisches Patentamt
European Patent Office
Office européen des brevets

(11) **EP 2 862 941 A1**

(12) **EUROPÄISCHE PATENTANMELDUNG**

(43) Veröffentlichungstag:
**22.04.2015 Patentblatt 2015/17**

(51) Int Cl.:
*C12Q 1/18* (2006.01) *C12Q 1/34* (2006.01)

(21) Anmeldenummer: **13004963.8**

(22) Anmeldetag: **17.10.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **VI-Diagnostics GmbH**
**04103 Leipzig (DE)**

(72) Erfinder: **Semjonov, Valeri, M.**
**210032 Witebsk (BY)**

(74) Vertreter: **Nenning, Peter**
**Riesaer Strasse 164**
**04319 Leipzig (DE)**

Bemerkungen:
Geänderte Patentansprüche gemäss Regel 137(2) EPÜ.

(54) **Verfahren zur quantitativen Bestimmung der beta-Lactamase-Aktivität in Körperflüssigkeiten und Bakteriensuspensionen**

(57)     Die Erfindung betrifft die Erfassung und quantitative Bewertung der ß-Lactamase-Aktivität in biologischen Flüssigkeiten (z.B. Blutserum, Sputum, Cerebrospinalflüssigkeit, Speichel, Urin, sowie von Pleura- und Aszitesflüssigkeit) und bakterieller Suspensionen mit dem Ziel einer deutlichen Verbesserung der antibakteriellen Therapie für Patienten mit Infektionskrankheiten bakterieller Natur.

Die Aufgabe der Erfindung ist die Entwicklung eines quantitativen labordiagnostischen Verfahrens zur Bestimmung der Therapieresistenz gegenüber β-Lactam-Antibiotika. Die Erfindung kann sowohl zur Erfassung bakterieller ß-Lactamasen, als auch zur Bewertung der durch endogene Makroorganismus-Faktoren bedingten (sogenannte "biologische") Resistenz benutzt werden.

Das Verfahren basiert auf der Erkenntnis, dass bei Wechselwirkung mit beliebigen Faktoren, die eine ß-Lactamase-Aktivität aufweisen, eine hydrolytische Spaltung der β-Lactam-Verbindung im Molekül des synthetischen Antibiotikums Nitrocefin auftritt, was zu einer bathochromen Verschiebung im Chromophorsystem von Nitrocefin führt.

EP 2 862 941 A1

## Beschreibung

Die Erfindung gehört in den Bereich der Medizin (Infektiologie, klinische Mikrobiologie) und kann zur qualitativen und quantitativen Bestimmung der ß-Lactamase-Aktivität in Bakteriensuspensionen und Körperflüssigkeiten wie Blutserum, Sputum, Cerebrospinalflüssigkeit, Speichel, Urin, sowie von Pleura- und Aszitesflüssigkeit benutzt werden. Als mögliche Matrizes eignen sich alle biologischen Flüssigkeiten, aus denen man ein optisch transparentes Filtrat erhalten kann.

Bei ß-Lactamasen handelt es sich um Enzyme, die die Amid-Bindung innerhalb des Grundgerüstes der ß-Lactam-Antibiotika (ß-Lactam-Ring) hydrolytisch spalten können.

Die zunehmende Resistenz von Bakterien gegenüber Antibiotika ist aktuell eines der wichtigsten Probleme der Infektiologie. Bis auf wenige Ausnahmen zeigen nahezu alle bekannten bakteriellen Erreger von Infektionskrankheiten mehr oder weniger stark ausgeprägte Resistenzen gegenüber antibakteriellen Präparaten.

Antibiotika der ß-Lactam-Gruppe gelten als die erfolgreichsten antibakteriellen Präparate seit dem Beginn der Antibiotikatherapie [30]. Allerdings hat die Resistenz der Bakterien gegenüber ß-Lactam-Antibiotika in den letzten 60 Jahren kontinuierlich zugenommen, sodass befürchtet wird, dass ß-Lactame-Antibiotika zukünftig nicht mehr in der Lage sind, schwerwiegenden bakteriellen Infektionen zu therapieren [13]. Die Resistenz gegenüber ß-Lactam-Antibiotika und ß-Lactamase-Inhibitoren ist ein stetig wachsendes Problem, das zur Verringerung der klinischen Effektivität von Wirkstoffen führt, die die Basis der Antibiotikatherapie bilden [20].

Die konnatale oder erworbene Fähigkeit ß-Lactamasen zu produzieren wird als Hauptmechanismus der Resistenz von Bakterien gegenüber ß-Lactam-Antibiotika angesehen. Nahezu alle bekannten krankheitserregenden Mikroorganismen können β-Lactamasen synthetisieren [32, 33, 35].

Bis zur heutigen Zeit wurde die Resistenz krankheitserregender Bakterien gegenüber Antibiotika lediglich als eine Anpassungsreaktion der Mikroorganismen betrachtet. Dabei wird allerdings nicht berücksichtigt, dass der menschliche Organismus selbst die Fähigkeit besitzt, Antibiotika zu inaktivieren. Hierfür werden verschiedene Mechanismen genutzt, um das als Fremdstoff erkannte Antibiotikum wieder zu eliminieren. Dem menschlichen Organismus fehlen Enzyme analog der bakteriellen ß-Lactamase [17], trotzdem kann hämolysiertes Blut die 3-Acetoxymethyl-cephalosporine (Cefalotin, Cefotaxim) durch Deacylierung der 3-Acetoxymethyl-Gruppe [36] inaktivieren. Es ist ferner bekannt, dass Carbapeneme (speziell Imipenem) durch die renale Dehydropeptidase zerstört werden können, weshalb Imipenem in Kombination mit Cilastatin, einem Inhibitor der renalen Dehydropeptidase, verabreicht wird [25]. Zudem können Analoga der Carbapeneme von humanen Serumalbuminen zerstört werden können. Die Globulinfraktion besitzt hingegen keine solche Aktivität [14].

Das Phänomen der eigenen (endogenen) ß-Lactamase-Aktivität von Humanblut ist bereits länger bekannt. Wissenschaftler der Glaxo Research Ltd., die die Eigenschaften des von ihnen synthetisierten chromogenen Cephalosporins Nitrocefin untersuchten, beschrieben 1972 einen signifikanten Zerfall der ß-Lactam-Struktur von Nitrocefin, unter anderem unter Einwirkung von humanen Blutserum. Dabei wurde gezeigt, dass diese Eigenschaft in erster Linie durch die Albumin-Fraktion vermittelt wird [28]. Es erfolgten allerdings keine weitergehenden Untersuchungen, da die Reaktion als unspezifisch betrachtet wurde. Erst 1994 wurde durch Nerli et al. das Phänomen eines intensiven Zerfalls von Nitrocefin unter Einwirkung von humanen Serumalbumin erneut beschrieben. Dieser Zerfall konnte jedoch nicht für andere Antibiotika der Cephalosporin-Reihe (Ceftriaxon, Cefoperazon und Cefsulodin) gezeigt werden. Die klinische Bedeutung des Phänomens wurde nicht untersucht, daher blieb das Auftreten ungewöhnlich hoher endogener ß-Lactamase-Aktivitäten in Humanblut von der wissenschaftlichen Gemeinschaft weiter unbeachtet. 2007 wurde das Phänomen des intensiven Zerfalls von Nitrocefin in Gegenwart von humanen Blutserum von Semenov et al. [7] beschrieben. Semenov et al. (2009) stellten fest, dass die ß-Lactamase-Aktivität des Blutserums überwiegend durch humanes Serumalbumin und nur in geringem Maße durch polyklonales IgG vermittelt wird [11].

Das Phänomen der Bildung von Immunglobulinen im menschlichen Organismus ist dabei gesondert zu betrachten. Die ß-Lactamase-Aktivität der Immunglobuline beruht auf ihren "Abzymeigenschaften" (katalytische Antikörper). Verschiedene Untersuchungen haben gezeigt, dass Antikörper in der Lage sind, in Ligand-Rezeptor-Beziehungen jeglicher Natur einzugreifen [3, 31] und sie dementsprechend auch die Funktion von Enzymen einnehmen können [18, 19]. In einer Reihe von Untersuchungen konnte für Antikörper eine enzymähnliche Aktivität gezeigt werden [9, 22, 23]. Heute geht man davon aus, dass sich die "Abzyme" im Organismus in natürlichen Umgebung als einer der Bausteine des Jerne-Immunnetzwerkes bilden [8, 21].

Bereits früher wurde experimentell gezeigt, dass bei Infektionskrankheiten, die durch Penicillinase-produzierende Erreger verursacht wurden (speziell bei durch P. aeruginosa ausgelöster Pneumonie [15]), die Bildung von Antikörpern gegen bakterielle Penicillinasen möglich ist. Laut Theorie des Jerne-Immunnetzwerkes, findet dabei eine Bildung sekundärer Antikörper statt [24, 34]. Mit hoher Wahrscheinlichkeit ist anzunehmen, dass solche Antikörper ß-Lactame hydrolytisch spalten können. Die Möglichkeit der Bildung von Abzymen mit Penicillinase-Aktivität, wurde in vivo (bei der Immunisierung von Mäusen mit Penicillinase) in verschiedenen Arbeiten gezeigt [12, 16, 17, 29]. Untersuchungen von Semenov et al. haben das Phänomen der Antikörper-Bildung mit signifikanter Penicillinase-Aktivität bei Patienten mit Shigellose erstmalig in vivo nachgewiesen [5, 10].

Auf Grundlage der oben genannten Fakten ergeben sich neue Ansätze zur Erklärung einiger oft beobachteter Phänomene. So wird bei einer Reihe von Infektionskrankheiten in vivo eine (klinische) Ineffizienz verschiedener ß-Lactam-Antibiotika beschrieben, die in vitro wirksam gegen die entsprechenden bakteriellen Erreger waren [2].

Der Erfindung liegt die wissenschaftliche Erkenntnis zugrunde, dass im menschlichen Organismus Faktoren präsent sind, die den Abbau und die Eliminierung von ß-Lactam-Antibiotika aus dem Organismus beschleunigen und erleichtern können. Dabei handelt es sich vor allem um humanes Serumalbumin und katalytische Antikörper ("Abzyme") mit ß-Lactamase-Aktivität. Dieses Phänomen wird als "biologische" Resistenz gegenüber ß-Lactam-Antibiotika bezeichnet und beschreibt die verminderte Effizienz einer Therapie mit diesen Präparaten durch ein individuell hohes endogenes Niveau der ß-Lactamase-Aktivität des biologischen Organismus, bedingt durch endogene Faktoren der Makroorganismen. Somit kann sich das Phänomen der "biologischen" Resistenz gegenüber ß-Lactam-Präparaten mit der "mikrobiellen" Resistenz der bakteriellen Erreger als Ergebnis einer Produktion verschiedener ß-Lactamasen überlagern und synergistisch beeinflussen.

## Defizite des Standes von Wissenschaft und Technik

Es ist bisher keine Methode zur quantitativen Bestimmung der endogenen Resistenzlage gegenüber ß-Lactam-Antibiotika bekannt. Zwar existieren Methoden zur Bewertung des Resistenzniveaus pathogener Mikroorganismen gegenüber ß-Lactam-Antibiotika, jedoch sind diese kompliziert, teuer und besitzen eine niedrige Reproduzierbarkeit. Die Analyse mit diesen Methoden nimmt zudem viel Zeit in Anspruch (durchschnittlich mehr als einen Tag) und bedingt die vorherige Herstellung einer Monokultur des vermeintlichen bakteriellen Krankheitserregers.

Der **Prototyp der deklarierten Methode** ist ein Disk-Set mit Nitrocefin von Beckton Dickinson BBL (Katalognummer 231650) [1], was zur Durchführung des Disk-Diffusionsverfahrens bestimmt ist. Diese Methode beinhaltet folgende Schritte: 1) Vorselektion der Monokultur des Krankheitserregers und dessen Identifikation; 2) Abimpfung der Monokultur in das standardmäßige Nährmedium (normalerweise Müller-Hinton-Agar), auf dessen Oberfläche mit Nitrocefin imprägnierte Cefinase-Disks aufgebracht werden; 3) visuelle Erfassung der Farbänderung der mit Nitrocefin imprägnierten Cefinase-Disks nach einer Inkubationszeit von 1-60 min.

Als **Eigenschaften** der Prototypmethode gelten:

1) Benu tzung der mit Nitrocefin imprägnierten Cefinase-Disks (Standardgröße)
2) Zur Bewertung der ß-Lactamase-Aktivität des Substrates wird die visuelle Erfassung der Farbänderung von Nitrocefin aufgrund der hydrolytischen Spaltung der ß-Lactam-Struktur benutzt; die Dauer der Analyseprozedur beträgt zwischen 1 und 60 min

Als **Nachteile** der Prototypmethode gelten:

1) Erfassung der Farbänderung der mit Nitrocefin imprägnierten Cefinase-Disks erfolgt lediglich visuell
2) ß-Lactamase-Aktivität wird nur aus der Monokultur der Bakterien bestimmt; hierfür muss diese zunächst aus dem biologischen Material hergestellt werden, wobei die Vorselektion der Monokultur nicht immer erfolgreich und zudem zeitaufwendig ist (bis zu 96 h)
3) Status der individuellen "biologischen" Resistenz gegenüber ß-Lactam-Antibiotika kann nicht beurteilt werden [1], da diese nicht mit der Prototypmethode erfasst werden kann

## Aufgabe der Erfindung

Die Aufgabe der Erfindung besteht in der Entwicklung einer einfachen, sicheren, zuverlässigen und reproduzierbaren Labormethode, mit welcher der Status der ß-Lactamase-Aktivität in Körperflüssigkeiten (Blutserum, Sputum, Cerebrospinalflüssigkeit, Speichel, Urin, sowie Pleura- und Aszitesflüssigkeit) und in bakteriellen Suspensionen qualitativ und quantitativ bestimmt werden kann. Sinn dieser diagnostischen Methode zur Bestimmung der ß-Lactamase-Aktivität ist eine mögliche Korrektur der empirischen Antibiotikatherapie bei Patienten mit bakteriellen Infektionskrankheiten.

Das Wesen der Erfindung besteht darin, dass biologische Flüssigkeiten oder bakterielle Suspensionen mit einer vorgegebenen Menge an Nitrocefin ("Chromogensubstrat" für ß-Lactamasen) versetzt und bei 37°C 30 min (Blutserum) bzw. 120 min (Sputum, Cerebrospinalflüssigkeit, Speichel, Urin, Pleura- und Aszitesflüssigkeit) inkubiert werden. Die Kontrollproben enthalten anstelle von Nitrocefin destilliertes Wasser. Als Positivkontrolle (vollständiger Zerfall von Nitrocefin) wird ein Gemisch aus Nitrocefin und Penicillinase eingesetzt, die Negativkontrolle (Blank) besteht aus Nitrocefin und destilliertem Wasser. Nach Abschluss der Inkubation erfolgt die Quantifizierung des Farbumschlages der Proben mittels Plattenphotometer (Microplate Reader), wobei der Anteil des in jeder Probe zerfallenen Nitrocefins erfasst wird. Mit dieser Methode kann ohne Herstellung einer Monokultur des bakteriellen Erregers festgestellt werden, ob eine vermehrte bakterielle ß-Lactamase-Produktion erfolgt. Ferner wird die endogene ("biologische") Resistenzlage erfasst.

Nitrocefin ((7*R*)-3-((*E*)-2,4-Dinitrostyryl)-7-(2-thienylacetamido)-3-cephem-4-carbonsäure) ist ein synthetisches Cephalosporin mit chromogenen Eigenschaften. Wird die Amid-Bindung des $\beta$-Lactam-Ring hydrolytisch gespalten, tritt eine bathochrome Verschiebung des Absorptionsmaximums von 390 auf 486 nm ein. Dies ist durch einen Farbumschlag von gelb nach orangerot sichtbar. Nitrocefin eignet sich daher als chromogenes Substrat zum kolorimetrischen Nachweis von ß-Lactamasen und zur spektralphotometrischen Detektion der ß-Lactamase-Aktivität.

Die NWG der Assayreaktion wurde mit 0,0004 (0,000381) mg/mL Nitrocefin bestimmt (n=48). Dies entspricht einer Bestimmungsgrenze von 0,0019 (0,001905) mg/mL Nitrocefin. Die Werte wurden für das Plattenphotometer iMark (BioRad, US) ermittelt. Für andere Geräte kann es geringfügige Abweichungen geben.

Die standardmäßig eingesetzte Penicillinase-Konzentration der Positivkontrolle ist mit 2000 U ausreichend hoch, da bereits ab 1000 U keine Änderung der ß-Lactamase-Aktivität mehr vorhanden war.

Sowohl die Intra-Assay-Reproduzierbarkeit, als auch die Inter-Assay-Reproduzierbarkeit konnten mit Variationskoeffizienten von 3,52% bzw. 3,40% belegt werden.

**Das Verfahren lässt sich in folgende Schritte gliedern:**

**Gewinnung der zu untersuchenden Matrices**

Blutserum erhält man aus frischem Vollblut, dass bei 4°C 4-6 h lang (Bildung des Fibrinklumpens/Blutkuchens) aufbewahrt und anschließend 15 min lang bei 3000 U/min zentrifugiert wurde. Das so gewonnene Blutserum kann bis zur Durchführung des Assays bei -20°C aufbewahrt. Einmal zur Durchführung des Assays aufgetaute Proben dürfen nicht weiter verwendet werden.

Sputum kann durch eine Fibrobronchoskopie gewonnen oder aus dem Intubationstubus entnommen werden. Nach der bakteriologischen Untersuchung werden die Sputumproben in Eppendorf-Reaktionsgefäßen bei -20°C aufbewahrt und vor dem Experiment einmalig aufgetaut. Die Vorbereitung der Sputumproben ist nachfolgend beschrieben:

1) Behandlung im Ultraschallbad 30 min lang bei 37°C zur Desintegration dichter Sputum- und Eiterklümpchen
2) Vortexen (5 min)
3) Zentrifugation (12000 U/min, 5 min).

Alle weiteren Untersuchungen erfolgen mit dem Überstand; der Niederschlag wird verworfen. Bei zu geringem Probenvolumen werden 0,5 mL physiologische Kochsalzlösung hinzugefügt und die Probe erneut wie oben beschrieben aufgearbeitet.

Cerebrospinalflüssigkeit (CSF; Liquor) wird durch eine Lumbalpunktion entnommen. Die CSF-Proben werden in Eppendorf-Reaktionsgefäßen bei -20°C aufbewahrt und vor der Untersuchung einmalig aufgetaut.

Speichel wird nüchtern oder 30 Minuten nach der Spülung der Mundhöhle mit 0,9%tiger NaCl-Lösung mit einer sterilen Pipette entnommen. Nach dem Zentrifugieren (7000 U/min, 5 min) der Speichelproben wird der klare Überstand in Eppendorf-Reaktionsgefäßen bei - 20°C aufbewahrt, vor der Untersuchung einmalig aufgetaut und erneut zentrifugiert. Ist das Volumen des Überstandes geringer als 2 mL, wird mit steriler physiologischer NaCl-Lösung auf 2 mL aufgefüllt.

Urinproben (Mittelstrahlurin) werden in Eppendorf-Reaktionsgefäßen bei -20°C aufbewahrt und vor der Untersuchung einmalig aufgetaut.

Bakteriensuspensionen werden aus frischen Reinkulturen der bakteriellen Erreger hergestellt. Die Isolierung und Identifizierung der Mikroorganismen wird nach allgemeingültigen Standards durchgeführt [4]. Die Inkubation erfolgt 24 h lang bei 37°C auf Schrägagar in Kolben (Endo- oder Ressel-Agar für Enterobakterien und für Pseudomonas aeruginosa; dotter-salinarer Agar für Staphylokokken; Müller-Hinton oder Lipovitellin-Salt-Mannitol Agar für S. aureus). Nach Zugabe von 2-3 mL steriler physiologischer Kochsalzlösung werden die Kolben geschüttelt bis die Flüssigkeit über dem Agar trüb wird. Diese trübe Flüssigkeit wird entnommen und falls erforderlich mit steriler physiologischer Kochsalzlösung auf eine optische Dichte von $0,125\pm0,03$ eingestellt ($\lambda$=625 nm, Schichtdicke 1 cm). Die Bakteriensuspensionen werden in Eppendorf-Reaktionsgefäßen bei -20°C aufbewahrt und vor der Untersuchung einmalig aufgetaut. Zur Verbesserung der analytischen Sensitivität wird empfohlen, die Bakteriensuspensionen vor der Untersuchung zunächst mindestens 15 min lang bei -20°C einzufrieren und anschließend 5-7 min lang bei 37°C in einem Thermostat aufzutauen.

**Durchführung des BIOLACTAM-Assays**

Herstellen der Lösungen:

- Phosphatuffer-Lösung (PB; pH 7,4): 4,6 mL destilliertes Wasser ($H_2O$) werden zu Vial 2 gegeben und vollständig gelöst

- Stammlösung des Chromogensubstrates ("Chromogen"): 500,0 µL PB werden zu Vial 1 gegeben und vollständig gelöst (1 Monat bei -18°C lagerbar)
- Katalyselösung ("Penicillinase"): 100 µL $H_2O$ werden zu Vial 3 gegeben und vollständig gelöst

Bestimmung der ß-Lactamase-Aktivität:

(A) Test von Blutserum:

- 4,0 mL PB werden zur Chromogen-Stammlösung gegeben (die Konzentration der Nitrocefin-Lösung beträgt dann 0,028 mg/mL); die Lösung muss innerhalb von 1 Tag verbraucht werden
- die entsprechenden Volumina (siehe Tabelle 1) werden in der Reihenfolge von rechts nach links in die Kavitäten geben und 30 min lang bei 37°C inkubieren **Tabelle 1** Volumina der Reagenzien / Kavität (jeweils 200 µL)

| Bezeichnung | Blutserum (µL) | $H_2O$ (µL) | Chromogen (µL) | Penicillinase (µL) |
|---|---|---|---|---|
| Testprobe (T) | 20 | - | 180 | - |
| Kontrollprobe (C) | 20 | 180 | - | - |
| Blank (B) | - | 20 | 180 | - |
| Positivkontrolle (P) | - | - | 180 | 20 |

(B) Test von anderen Körperflüssigkeiten und Bakteriensuspensionen:

- 2,0 mL PB werden zur Chromogen-Stammlösung geben (die Konzentration der Nitrocefin-Lösung beträgt dann 0,05 mg/mL); die Lösung muss innerhalb von 1 Tag verbraucht werden

- die entsprechenden Volumina (siehe Tabelle 2) werden in der Reihenfolge von rechts nach links in die Kavitäten geben (der Gesamtgehalt an Nitrocefin in 200 µL Reaktionslösung ist identisch der Bestimmung in Blutserum); 120 min lang bei 37°C inkubieren

**Tabelle 2** Volumina der Reagenzien / Kavität (jeweils 200 µL)

| Bezeichnung | Körperflüssigkeit (µL) | $H_2O$ (µL) | Chromogen (µL) | Penicillinase (µL) |
|---|---|---|---|---|
| Testprobe (T) | 100 | - | 100 | - |
| Kontrollprobe (C) | 100 | 100 | - | - |
| Blank (B) | - | 100 | 100 | - |
| Positivkontrolle (P) | - | 80 | 100 | 20 |

Messung der optischen Dichten (OD) und Berechnung der Ergebnisse:

Die OD werden für die Testprobe und die Kontrollen mit einem Plattenphotometer (Plate Reader) bei 492 (oder 505) nm gegen Luft gemessen und die ß-Lactamase-Aktivität der Probe mit folgender Formel berechnet.

$$A_{bl}[\%] = \frac{(OD_T - OD_C) - (OD_B - OD_E)}{OD_P - OD_E} \times 100$$

$A_{bl}$: β-Lactamase-Aktivitätsniveau der Testproben [%]; $OD_T$: Mittelwert der OD der Testproben;

$OD_C$: Mittelwert der OD der Kontrollproben; $OD_B$: Mittelwert der OD der Blankwerte;
$OD_P$: Mittelwert der OD der Positivkontrollen; $OD_E$: Mittelwert der OD von leeren Kavitäten (Hintergrund).

Mit Hilfe einer Kalibrierungskurve kann der erhaltene Wert an ß-Lactamase-Aktivität [%] in Penicillinase-Einheiten [U] umgerechnet werden.

**Beispiele**

Die Erfindung soll anhand folgender klinischer Anwendungsbeispiele näher erläutert werden.

**Untersuchung von Blutserum**

Die Untersuchung beinhaltete 419 Testproben. Es wurden zwei Subgruppen (Patientenkollektiv A und B) gebildet. Patientenkollektiv A bestand aus Patienten mit bakteriellen Infektionskrankheiten, die in der Regel initial mit ß-Lactam-Antibiotika behandelt worden waren (n=203). Hierzu zählten die folgenden Patienten mit Erysipel (n=53), bakterieller Pneumonie (n=62), bakterieller Meningitis (n=10) und bakteriell-eitriger Tonsillitis (n=78). Patientenkollektiv B (n=216) beinhaltete Patienten mit nicht-bakteriellen Infektionskrankheiten (virale Meningitis, n=9; Influenza A H1N1, n=32), Patienten mit Lungentuberkulose (n=63) und Patienten mit traumatologischen Pathologien ohne Merkmale von Infektionskrankheiten (n=61).

Die Mittelwerte (MW) und Mediane der gemessenen ß-Lactamase-Aktivitäten für die entsprechenden Patientengruppen der Patientenkollektive A und B wurden in Tabelle 3 und Tabelle 4 zusammengefasst.

**Tabelle 3** ß-Lactamase-Aktivität der untersuchten Blutserumproben der Patientengruppen von Patientenkollektiv A

| Patientengruppe | n | MW | 95% CI | min | max | 25‰ | Median | 75‰ |
|---|---|---|---|---|---|---|---|---|
| Erysipel | 53 | 52,3 | 49,2...55,5 | 20,9 | 71,7 | 46,4 | 54,9 | 61,2 |
| bakterielle Pneumonie | 62 | 55,8 | 52,8...58,7 | 0,0 | 66,6 | 55,5 | 59,6 | 61,3 |
| bakterielle Meningitis | 10 | 50,8 | 38,5...63,1 | 19,6 | 66,6 | 38,8 | 57,9 | 63,0 |
| bakteriell-eitrige Tonsillitis | 78 | 61,1 | 59,6...62,6 | 43,7 | 96,7 | 57,6 | 61,2 | 64,3 |

**Tabelle 4** ß-Lactamase-Aktivität der untersuchten Blutserumproben der Patientengruppen von Patientenkollektiv B

| Patientengruppe | n | MW | 95% CI | min | max | 25‰ | Median | 75‰ |
|---|---|---|---|---|---|---|---|---|
| Infektion mit Adenoviren | 51 | 62,9 | 61,5...64,2 | 53,1 | 71,5 | 59,4 | 62,8 | 66,1 |
| virale Meningitis | 9 | 63,1 | 60,0...66,3 | 57,1 | 69,9 | 60,2 | 64,6 | 65,2 |
| Influenza A (H1N1) | 32 | 54,3 | 50,2... 58,3 | 26,7 | 70,9 | 49,1 | 56,7 | 62,3 |
| Patienten mit traumatologischen Pathologien | 61 | 60,4 | 59,0...61,8 | 45,2 | 70,0 | 56,4 | 60,1 | 64,5 |
| Lungentuberkulose | 63 | 66,0 | 64,2...67,8 | 42,7 | 99,2 | 63,5 | 65,6 | 68,6 |

Der Vergleich der ß-Lactamase-Aktivitäten von Blutserumproben der verschiedenen Subgruppen lieferte keine nennenswerten Unterschiede.

Ein Zusammenhang zwischen ß-Lactamase-Aktivität, Therapieerfolg und Medikation wurde an Patientenkollektiv A weiter untersucht. Die Anzahl der eingeschlossenen Patienten wurde dabei auf n=186 begrenzt, um eine identische Probenzahl in den beiden Gruppen mit niedriger beziehungsweise hoher ß-Lactamase-Aktivität zu gewährleisten. Das für die weiteren Untersuchungen verwendete Probenmaterial setzte sich daher folgendermaßen zusammen: Erysipel (n=48), bakterielle Pneumonie (n=60), bakterielle Meningitis (n=7) und bakteriell-eitrige Tonsillitis (n=71).

Eine weitere Aufteilung des Patientenkollektivs A erfolgte in Abhängigkeit eines vorgenommenen Therapiewechsels. Dies war bei 86 Patienten (46,8%) der Fall. Ein Patient mit Erysipel wurde bei der Analyse der Daten nicht berücksichtigt, da aus der Anamnese eine allergische Reaktion gegen ß-Lactam-Antibiotika bekannt war.

Innerhalb dieser Subgruppen der Patienten mit Therapiewechsel (n=86) bzw. ohne Therapiewechsel (n=99) wurde auf der Basis des Cut-off-Wertes von 68,2% zwischen einer basal hohen ß-Lactamase-Aktivität (≥68,2%) und einer entsprechend niedrigeren ß-Lactamase-Aktivität (<68,2%) unterschieden (siehe Tabelle 5).

**Tabelle 5** Vergleich der ß-Lactamase-Aktivität innerhalb der Subgruppen Patienten mit/ohne Therapiewechsel

| Therapiewechsel | β-Lactamase-Aktivität $\geq$68,2% | β-Lactamase-Aktivität <68,2% |
|---|---|---|
| nein | 55 (55,6%; 95% CI: 45,8...65,3) | 44 (44,4%; 95% CI: 34,7...54,2) |
| ja | 38 (43,7%; 95% CI: 33,4...54,1) | 49 (56,3%; 95 CI: 45,9...66,7) |

Der Chi-Quadrat-Test ergab keinen signifikanten Unterschied (p=0,11) zwischen der Anzahl an Patienten mit hoher und niedriger ß-Lactamase-Aktivität zwischen den Subgruppen mit oder ohne Therapiewechsel.

Zwischen den Subgruppen mit/ohne Therapiewechsel wurden signifikante Unterschiede (p<0,001; Mann-Whitney-U-Test (MWU-Test)) hinsichtlich folgender, den Krankheitsverlauf beschreibender Parameter festgestellt (siehe Tabelle 6): Hospitalisierungsdauer, Schweregrad der Erkrankungen, maximale Höhe des Fiebers und Dauer der Fieberperiode.

**Tabelle 6:** Vergleich des Verlaufs der Infektionskrankheiten der untersuchten Patienten. Signifikante Unterschiede ergaben sich für sämtliche Parameter zwischen den Gruppen mit bzw. ohne Therapiewechsel

| Parameter | Therapiewechsel | MW (95% CI) | Median (25;75 ‰) |
|---|---|---|---|
| HD (Tage) | nein | 9,9 (9,0...10,9) | 9 (7;11) |
| | ja | 15,3 (13,4...17,3) | 12 (9;17) |
| ESG | nein | 1,7 (1,6...1,8) | 2 (1;2) |
| | ja | 2,1 (1,9...2,2) | 2 (2;2) |
| $F_{max}$ (°C) | nein | 37,9 (37,7...38,1) | 37,8 (37,2;38,6) |
| | ja | 38,4 (38,2...38,5) | 38,2 (37,6;39,0) |
| $F_d$ (Tage) | nein | 2,3 (1,9...2,8) | 2 (1;3) |
| | ja | 5,1 (4,1...6,1) | 4 (2;6) |
| *HD: Hospitalisierungsdauer; ESG: Schweregrad der Erkrankungen (Einschätzung von klinischen Experten); $F_{max}$: maximale Höhe des Fiebers; $F_d$: Dauer der Fieberperiode* | | | |

Patienten, bei denen ein Therapiewechsel erforderlich wurde, hatten im Durchschnitt höheres Fieber (38,4°C vs. 37,9°C; p=0,0013; MWU-Test) und eine längere Fieberperiode (5,1 vs. 2,3 Tage; p<0,0001; MWU-Test). Dazu war die Hospitalisierungsdauer mit durchschnittlich 15,3 Tagen im Vergleich zu 9,9 Tagen deutlich länger (p<0,0001; MWU-Test). Außerdem wurden die Krankheitsverläufe von den klinischen Experten subjektiv als schwerer beurteilt.

Der Vergleich der durchschnittlichen Hospitalisierungsdauer innerhalb der Subgruppen Patienten mit/ohne Therapiewechsel in Abhängigkeit der ß-Lactamase-Aktivität ($\geq$68,2% vs. <68,2%) wurde in Tabelle 7 dargestellt. Dabei wurde ein signifikanter Unterschied von 4,6 Tagen (p=0,003; MWU-Test) bei Patienten mit hoher ß-Lactamase-Aktivität festgestellt, während sich die Verweildauer im Krankenhaus von Patienten mit niedriger ß-Lactamase-Aktivität durch einen Wechsel der Antibiotikatherapie nur unwesentlich verbesserte (0,5 Tage; p=0,44; MWU-Test).

**Tabelle 7:** Vergleich der durchschnittlichen Hospitalisierungsdauer (in Tagen) in Abhängigkeit der ß-Lactamase-Aktivität innerhalb der Subgruppen Patienten mit/ohne Therapiewechsel

| Hospitalisierungsdauer (in Tagen) | | | | |
|---|---|---|---|---|
| | 10,2 (95% CI: 8,8...11,6) | 9,7 (95% CI: 8,3...11,1) | 17,3 (95% CI:14,6...20,1) | 12,7 (95% CI:10,2...15,3) |
| | p=0,44 | | p=0,003 | |
| β- Lactamase-Aktivität | <68,2% | | $\geq$68,2% | |
| Therapiewechsel | nein | ja | nein | ja |

In der Subgruppe mit Wechsel der ursprünglichen Antibiotikatherapie innerhalb des Patientenkollektivs mit bakteriellen Infektionskrankheiten konnte ein signifikanter Zusammenhang zwischen einer hohen ß-Lactamase-Aktivität im Blutserum und der Effektivität des Therapiewechsels durch Antibiotika anderer Wirkstoffgruppen festgestellt werden.

In der Subgruppe von Patienten mit hoher ß-Lactamase-Aktivität im Blutserum führte der Therapiewechsels zu Antibiotika anderer Wirkstoffgruppen (oder Inhibitor-geschützen ß-Lactamen) zu einer deutlichen Verkürzung der Krankenhausverweildauer im Vergleich zur Subgruppe von Patienten mit niedriger ß-Lactamase-Aktivität im Blutserum.

Ist die ß-Lactamase-Aktivität ≥70%, wird die Verordnung von Inhibitor-geschützen ß-Lactamen oder Antibiotika anderer pharmakologischer Gruppen empfohlen.

## Untersuchung von Cerebrospinalflüssigkeit

Die Untersuchung beinhaltete 208 CSF-Proben, die durch diagnostische Lumbalpunktionen von Patienten mit viraler (n=68) und bakterieller Meningitis (n=34) gewonnen wurden. Darüber hinaus wurden 73 CSF-Proben von Patienten, die aufgrund eines unbestätigten Verdachts auf infektiöse Läsionen des ZNS eine umfassende Differentialdiagnose unterzogen wurden, in die Untersuchung als Kontrollen eingeschlossen. 24 CSF-Proben stammten von Personen mit einer Subarachnoidalblutung von unterschiedlicher Schwere und Dauer.

Der Mittelwert der ermittelten ß-Lactamase-Aktivitäten der CSF-Proben war 15,9% (95% CI: 13,3...18,4) und der Median lag bei 9,2% (25‰-5,0, 75‰-19,9). Die Streuung der ß-Lactamase-Aktivitäten war relativ groß (0,0-97,8%).

Der Vergleich der ß-Lactamase-Aktivitäten der CSF-Proben innerhalb der Patientengruppen bakterielle Meningitis (n=43), virale Meningitis (n=68), Patienten mit Subarachnoidalblutung (n=24) und Patienten ohne Merkmale entzündlicher oder traumatischer Schädigungen des ZNS (n=73) wurde Tabelle 8 dargestellt.

**Tabelle 8** $\beta$-Lactamase-Aktivität [%] der untersuchten CSF-Proben (Mittelwerte und Mediane)

| Patientengruppe | n | MW | 95% CI | 25‰ | Median | 75‰ |
|---|---|---|---|---|---|---|
| bakterielle Meningitis | 43 | 19,4 | 13,6...25,1 | 4,1 | 13,1 | 28,2 |
| virale Meningitis | 68 | 14,9 | 10,4...19,4 | 6,0 | 9,4 | 15,3 |
| Patienten mit Subarachnoidalblutung | 24 | 27,2 | 18,4...35,9 | 16,5 | 21,2 | 32,1 |
| Patienten ohne ZNS-Schäden | 73 | 9,0 | 5,0...12,9 | 2,5 | 5,7 | 8,6 |

Die Ergebnisse des paarweisen Vergleichs der Mediane der ß-Lactamase-Aktivität in CSF mittels MWU-Test wurden in Tabelle 9 dargestellt. Mit Ausnahme des Vergleichs der ß-Lactamase-Aktivität in CSF von Patienten mit bakterieller vs. viraler Meningitis wurden für alle Patientengruppen signifikante Unterschiede festgestellt.

Die signifikant erhöhte ß-Lactamase-Aktivität in CSF von Patienten mit einer Subarachnoidalblutung wurde vermutlich durch einen deutlichen Anstieg von Serumalbumin hervorgerufen.

**Tabelle 9:** Ergebnisse des paarweisen Vergleichs der Mediane der $\beta$-Lactamase-Aktivitäten der CSF-Proben in den Patientengruppen (MWU-Test)

| Patientengruppe | bakterielle Meningitis | virale Meningitis | Patienten mit Subarachnoidalblutung | Patienten ohne ZNS-Schäden |
|---|---|---|---|---|
| bakterielle Meningitis | | p=0,31 | p<0,05 | p<0,001 |
| virale Meningitis | p=0,31 | | p<0,0001 | p<0,001 |
| Patienten mit Subarachnoidalblutung | p<0,05 | p<0,0001 | | p<0,0001 |
| Patienten ohne ZNS-Schäden | p<0,001 | p<0,001 | p<0,0001 | |

Im Allgemeinen deuten die Ergebnisse einer Korrelationsanalyse nach Spearman auf einen direkten Zusammenhang zwischen der $\beta$-Lactamase-Aktivität in CSF und der Ausprägung einer entzündlichen Reaktion der Hirnhaut hin.

Die Häufigkeitsverteilung im Histogramm der gemessenen ß-Lactamase-Aktivitäten der CSF-Proben (n=208) zeigt keine Normalverteilung, sondern ist nach links verschoben. Die Mehrheit der CSF-Proben (n=156; 75%) hatte nur eine geringe $\beta$-Lactamase-Aktivität (<20%). Nur 52 (25%) der CSF-Proben wiesen eine hohe $\beta$-Lactamase-Aktivität (≥20%) auf.

Da die Untersuchung der Effektivität der Antibiotika-Therapie nur Sinn bei Patienten macht, die aufgrund ihrer Diagnose oder vermuteten Diagnose Antibiotika erhielten, wurden das Patientenkollektiv der Studie auf eine Subgruppe von Patienten mit infektiöser ZNS-Pathologie, welche Antibiotika (n=74) erhielten, verkleinert. Darunter befanden sich 43 Patienten mit bestätigter und 31 Patienten mit vermuteter bakterieller Meningitis.

Mit einer Korrelationsanalyse nach Spearman wurde die Effektivität der antibakteriellen Therapie der Patienten mit neurologischen Infektionskrankheiten (nachgewiesene und vermutete Diagnose "bakterielle Meningitis"; n=74) in Abhängigkeit der β-Lactamase-Aktivität der entsprechenden CSF-Proben untersucht. Dabei wurden folgende Zusammenhänge ermittelt:

- eine schwache Korrelation der β-Lactamase-Aktivität und der Hospitalisierungsdauer ($r_s$=0,238; p=0,041; n=74)
- eine Korrelation der β-Lactamase-Aktivität und der maximalen Anzahl der gleichzeitig verschriebenen Antibiotika ($r_s$=0,388; p=0,003; n=74)
- eine Korrelation der β-Lactamase-Aktivität und der Verordnung von ß-Lactam-Reserveantibiotika (Carbapeneme und Cephalosporine der 3. Generation) ($r_s$=0,301; p=0,009; n=74)
- eine Korrelation zwischen der β-Lactamase-Aktivität und der Verordnung von Antibiotika anderer pharmakologischer Wirkstoffgruppen ($r_s$=0,283; p=0,015; n=74)
- eine Korrelation zwischen der β-Lactamase-Aktivität und der Gesamtzahl der während der Therapie verordneten Antibiotika ($r_s$=0,289; p=0,013; n=74).

Für Patienten mit nachgewiesener bakterieller Meningitis (n=43) wurden für die gleichen Parameter stärkere Zusammenhänge festgestellt.

Die Korrelationsanalyse nach Spearman ergab:

- eine deutliche Korrelation der β-Lactamase-Aktivität und der maximalen Anzahl der gleichzeitig verschriebenen Antibiotika ($r_s$=0,586; p=0,0001; n=43)
- eine Korrelation der β-Lactamase-Aktivität und der Verordnung von ß-Lactam-Reserveantibiotika (Carbapeneme und Cephalosporine der 3. Generation) ($r_s$=0,381; p=0,017; n=43)
- eine deutliche Korrelation zwischen der β-Lactamase-Aktivität und der Gesamtzahl der während der Therapie verordneten Antibiotika ($r_s$=0,522; p=0,001; n=43).

Zusammenfassend konnte ein Zusammenhang zwischen einer erhöhten ß-Lactamase-Aktivität in CSF (≥20%) und einer Reduktion der Effizienz der initialen Antibiotika-Therapie mit klassischen ß-Lactamen (Cephalosporine der 3. Generation oder Aminopenicilline, seltener Penicilline G) bei Patienten mit bakteriellen entzündlichen Schädigungen des ZNS gezeigt werden. Dies machte die Verordnung von Reserveantibiotika der ß-Lactam-Gruppe oder anderer pharmakologischer Wirkstoffgruppen (Aminoglykoside, Rifampicin, Chloramphenicol, Chinolone, Cotrimoxazol u.a.) erforderlich.

Für den Vergleich der klinischen Daten hinsichtlich des Schweregrades des Krankheitsverlaufs und der Effizienz der verordneten Antibiotika-Therapie wurde ein MWU-Test für die folgenden Subgruppen durchgeführt: Subgruppe 1 (ß-Lactamase-Aktivität der CSF-Proben <20%; n=53) und Subgruppe 2 (β-Lactamase-Aktivität der CSF-Proben ≥20%; n=21) (Tabelle 10).

**Tabelle 10:** Ergebnisse des MWU-Tests zum Vergleich von Surrogatparameter zwischen den Subgruppen mit hoher (≥20%) und niedriger (<20%) β-Lactamase-Aktivität in CSF (Mittelwert = MW)

| Parameter | Subgruppe 1 MW (95% CI) | Subgruppe 2 MW (95% CI) | p |
|---|---|---|---|
| Dauer der Antibiotikatherapie (Tage) | 16,5 (12,9...20,1) | 21,8 (14,2...29,3) | > 0,05 |
| Hospitalisierungsdauer (Tage) | 22,7 (19,2...26,2) | 29,3 (20,0...38,7) | > 0,05 |
| maximale Anzahl gleichzeitig verordneter Antibiotika | 1,3 (1,1...1,5) | 2,0 (1,7...2,3) | 0,0015 |
| Anzahl der Therapiewechsel | 0,8 (0,5...1,2) | 1,6 (0,9...2,3) | 0,043 |
| Verordnung von Reserveantibiotika aus der β-Lactam-Gruppe (%) | 22,6 (11,0...34,3) | 52,3 (29,1...75,7) | 0,047 |

(fortgesetzt)

| Parameter | Subgruppe 1 MW (95% CI) | Subgruppe 2 MW (95% CI) | p |
|---|---|---|---|
| Verordnung Antibiotika aus anderen Wirkstoffgruppen (%) | 35,8 (22,5...49,2) | 76,2 (56,3...96,1) | 0,0066 |
| Verordnung von Reserveantibiotika aus anderen Wirkstoffgruppen (%) | 30,2 (17,4...43,0) | 57,1 (34,1...80,2) | 0,072 |
| Gesamtzahl der verordenten Antibiotika | 2,2 (1,7...2,8) | 4,0 (3,0...5,0) | 0,0018 |

*Antibiotika zur Behandlung von infektiösen Erkrankungen des ZNS*

- *ß-Lactam-Antibiotika der 1. Wahl: Cephalosporine der 3. Generation, Aminopenicillin (Ampicillin); selten Benzylpenicillin (Penicillin G)*
- *Reserve-Antibiotika aus der ß-Lactam-Gruppe: Carbapeneme, Cephalosporine der 3. Generation; Inhibitor-geschützte ß-Lactame, die die Blut-Hirn-Schranke überwinden können (Amoxicillin/Clavulensäure oder Ticarcillin/Clavulensäure)*
- *Antibiotika der 1. Wahl aus anderen pharmakologischen Gruppen: Aminoglykoside, Fluorchinolone der 2. Generation (Ciprofloxacin, Norfloxacin)*
- *Reserve-Antibiotika aus anderen pharmakologischen Gruppen: Rifampicin, Chloramphenicol, Cotrimoxazol, Glycopeptide (Vancomycin), Oxazolidinone (Linezolid), Fluorchinolone der 3. und 4. Generation (Levofloxacin, Moxifloxacin, Sparfloxacin)*

Die Unterschiede im therapeutischen Ansatz implizieren, dass die Wahrscheinlichkeit eines Versagens der Antibiotika-Therapie bei Patienten mit einer relativ hohen ß-Lactamase-Aktivität in der CSF ($\geq$20%) durchschnittlich 1,76 (1,32; 2,31) mal höher war. Dadurch waren häufiger Therapiewechsel (Verordnung von Reserveantibiotika einschließlich stark wirksamen ß-Lactam-Antibiotika wie Carbapeneme oder Cephalosporine der 3. Generation) notwendig. Außerdem mussten mehr Antibiotika gleichzeitig verordnet werden und die Hospitalisierungsdauer war deutlich länger.

Für Parameter, die mit "ja oder nein" beantwortet werden können, wurde das Risikoverhältnis (*risk ratio,* RR) ermittelt (siehe Tabelle 10).

**Tabelle 11:** Risikoverhältnis (RR) für Parameter der Effektivität der Antibiotika-Therapie bei Patienten mit neurologischen Infektionskrankheiten

| Parameter | RR | 95% CI |
|---|---|---|
| 2 und mehr Therapiewechsel | 1,94 | 1,01...3,72 |
| Verordnung von Reserveantibiotika der ß-Lactam-Gruppe | 2,31 | 1,22...4,40 |
| Therapiebeginn mit Antibiotika anderer pharmakologischer Wirkstoffgruppen | 2,12 | 1,38...3,27 |
| Verordnung von Reserveantibiotika anderer pharmakologischer Wirkstoffgruppen | 1,89 | 1,09...3,29 |
| 3 oder mehr Antibiotika verordnet | 1,86 | 1,16...2,98 |
| 4 oder mehr Antibiotika verordnet | 2,33 | 1,28...4,25 |
| 5 oder mehr Antibiotika verordnet | 3,24 | 1,39...7,58 |
| 6 oder mehr Antibiotika verordnet | 5,89 | 1,68...20,65 |

Durch die Berechnung der Risikoverhältnisse wurde gezeigt, dass die Wahrscheinlichkeit eines Fehlschlagen der initialen ß-Lactam-Antibiotika-Therapie bei Patienten mit bakteriellen Infektionen des ZNS und einer hohen ß-Lactamase-Aktivität in CSF ($\geq$20%) durchschnittlich um 2,02 (1,86; 2,31) mal höher war.

Für einen Teil (n=24) der Patienten mit vermuteter und bakterieller Meningitis wurde eine zweite Lumbalpunktion nach 10-12 Tagen Behandlungsdauer durchgeführt. Für diese 24 CSF-Proben wurde neben dem Pleozytosegrad die ß-Lactamase-Aktivität erneut bestimmt und mit den Werten vor Beginn der Therapie verglichen (siehe Tabelle 11).

**Tabelle 11:** Vergleich der ß-Lactamse-Aktivität vor- und nach der Antibiotikatherapie

| CSF-Probe vor Therapiebeginn MW $\beta$-Lactamase-Aktivität $\pm$ SD (95% CI) | CSF-Kontrollprobe nach 10-12 Tagen MW $\beta$-Lactamase-Aktivität $\pm$ SD (95% CI) |
|---|---|
| 22,1 $\pm$ 19,8% (13,7...30,4) | 16,2 $\pm$ 20,1% (7,7...24,7) |

Dabei wurde eine signifikante Abnahme der ß-Lactamase-Aktivität beobachtet (Wilcoxon signed-rank Test; p=0,025). Dies lässt vermuten, dass die ß-Lactamse-Aktivität in CSF von Patienten mit Meningitits mit der Zeit abnimmt, was indirekt auf eine erfolgreiche antibiotische Therapie schließen lässt.

Bei einer ß-Lactamase-Aktivität in CSF $\geq$20% (unter Ausschluss einer Subarachnoidalblutung) ist ein Versagen der basalen Antibiotika-Therapie mit klassischen ß-Lactamen um das 1,8-2,3fache wahrscheinlicher. In diesem Falle wird empfohlen, auf stark wirksame ß-Lactam-Antibiotika wie Carbapeneme oder Cephalosporine der 3. Generation zu wechseln oder auf Antibiotika anderer pharmakologischer Wirkstoffgruppen wie Glycopeptide, Oxazolidinone, Chinolone, Aminoglykoside, Rifamycine oder Chloramphenicol zurückzugreifen. Eine Kombination von ß-Lactam-Antibiotika und Inhibitoren wie Clavulansäure ist nicht sinnvoll, da diese in der Regel die Blut-Hirn-Schranke nicht ausreichend passieren können.

**Untersuchung von Sputum**

Die Untersuchung beinhaltete 163 Testproben, die im Rahmen von routinemäßgen Fibrobronchoskopien von Patienten mit Atemwegserkrankungen gewonnen wurden. Dabei wurden folgende Diagnosen gestellt: bakterielle Pneumonie (n=124), chronisch obstruktive Lungenerkrankung (COPD; n=45), Lungenkarzinom (n=23), Sarkoidose (n=6) und Pulmonalarterienthrombembolie (n=3). Etliche Patienten litten an mehr als einer der genannten Erkrankungen, z.B. an Pneumonie und COPD (n=26), Pneumonie und Lungenkarzinom (n=5) sowie COPD und Lungenkarzinom (n=5).

Der Mittelwert der ß-Lactamase-Aktivität in den Sputumproben war 13,0% (95% CI: 10,5...15,5) und der Median der $\beta$-Lactamase-Aktivität des Sputums lag bei 7,3% (25‰-0; 75‰ - 83,4). Dabei variierte die gemessene ß-Lactamase-Aktivität von 0,0% bis zu 94,3%. Die Streuung der Mediane der ß-Lactamase-Aktivitäten innerhalb der Patientenkollektive bakterielle Pneumonie (n=124), fortgeschrittene COPD (n=19) und Lungenkarzinom (n=18) war gering (bakterielle Pneumonie: 7,1 %); fortgeschrittene COPD 8,8%; Lungenkarzinom 9,1%). Dies wurde durch einen Kruskal-Wallis-Test (p=0,97) und einen Median-Test (p=0,87) bestätigt.

Es wurde der Korreiationskoeffizient nach Spearman für einen Zusammenhang zwischen Ausmaß der ß-Lactamase-Aktivität und der Verordnung von Reserveantibiotika bestimmt. Hierbei wurden mit Levofloxacin und Doxycyclin zwei Antibiotika, die nicht aus der ß-Lactam-Gruppe stammen ($r_s$=0,235; p=0,003; n=163), sowie Reserveantibiotika aus der ß-Lactam-Gruppe (Imipenem, Cefepime und Amoxicillin/Clavulansäure) untersucht ($r_s$=0,287, p<0,001; n=163).

Der Zusammenhang war deutlicher bei Patienten mit unkomplizierten Pneumonien ($r_s$=0,322; p=0,0029; n=91), beziehungsweise für die Reserveantibiotika aus der ß-Lactam-Gruppe ($r_s$=0,286; p=0,0069; n=91). Zusätzlich wurde eine Korrelation zwischen der ß-Lactamase-Aktivität und der Anzahl der Therapiewechsel für die Reserveantibiotika Levofloxacin und Doxycyclin ($r_s$=0,231; p=0,027; n=91), sowie die Reserveantibiotika aus der ß-Lactam-Gruppe ($r_s$=0,214, p<0,042; n=91) untersucht.

Daraus lässt sich schließen, dass eine hohe ß-Lactamase-Aktivität in Sputum von Patienten mit bakteriellen Infektionen der oberen Luftwege mit einer geringen Effizienz der Behandlung mit ß-Lactam-Antibiotika einhergeht. Somit war oftmals die Notwendigkeit für die Verwendung von geeigneten Reserveantibiotika aus der ß-Lactam-Gruppe (Carbapeneme, Cephalosporine vierter Generation, Inhibitor-geschützte ß-Lactame) oder Reserveantibiotika anderer Antibiotikagruppen gegeben.

Das Histogramm der Häufigkeiten der gemessenen ß-Lactamase-Aktivitäten der Sputumproben (n=163) zeigt keine Normalverteilung, sondern ist nach links verschoben, d.h. die Mehrheit der Sputumproben zeigte keine relevante $\beta$-Lactam-Aktivität. Nur ein kleiner Teil (n=30; 18,4%) der Sputumproben zeigte eine hohe $\beta$-Lactamase-Aktivität ($\geq$ 20%). Der Median für die Anzahl der verordneten Antibiotika für diese Subgruppe des Patientenkollektivs lag bei 4 (25‰ - 3; 75‰ - 5).

Für den Vergleich der klinischen Labordaten hinsichtlich des Schweregrades des Krankheitsverlaufs und der Effizienz der verordneten Antibiotika-Therapie wurde ein MWU-Test für die folgende Subgruppen durchgeführt: Subgruppe 1 ($\beta$-Lactamase-Aktivität der Sputumproben <20%; n=133) und Subgruppe 2 ($\beta$-Lactamase-Aktivität der Sputumproben $\geq$20%; n=30) (Tabelle 12).

**Tabelle 12** Zusammenhang zwischen der ß-Lactamase-Aktivität im Sputum und dem Therapiefortgang (MWU-Test zum Vergleich der Subgruppen; Mittelwert = MW)

| Parameter | Subgruppe 1 MW (95% CI) | Subgruppe 2 MW (95% CI) | p |
|---|---|---|---|
| Anzahl der Therapiewechsel | 1,9 (1,8...2,0) | 2,3 (2,1...2,5) | 0,0019 |
| 3 und mehr Therapiewechsel | 0,1 (0,05...0,15) | 0,3 (0,13...0,47) | 0,0035 |
| Verordnung von Reserveantibiotika insgesamt (%) | 18,0 (11,4...24,7) | 36,7 (18,4...55,0) | 0,025 |
| Verordnung von Reserveantibiotika der $\beta$-Lactam-Gruppe (%) | 11,3 (5,8...16,7) | 26,7 (9,9...43,5) | 0,029 |
| Gesamtzahl der verordneten Antibiotika | 3,4 (3,2...3,6) | 3,9 (3,6...4,3) | 0,013 |
| 5 und mehr Antibiotika verordnet | 0,14 (0,08...0,19) | 0,30 (0,13...0,47) | 0,029 |

Die Unterschiede im therapeutischen Ansatz implizieren, dass die Wahrscheinlichkeit eines Versagens der Antibiotika-Therapie bei Patienten mit einer relativ hohen ß-Lactamase-Aktivität im Sputum (≥20%) signifikant um das 2-3fache größer war. Dadurch war ein Therapiewechsel (Verordnung von Reserveantibiotika) häufiger notwendig.
Für Parameter, die mit "ja oder nein" beantwortet werden können, wurde das Risikoverhältnis (*risk ratio,* RR) ermittelt (Tabelle 13).

**Tabelle 13** Risikoverhältnis (RR) für Parameter der Effektivität der Antibiotika-Therapie bei Patienten mit Erkrankungen der Lunge

| Parameter | RR | 95% CI |
|---|---|---|
| 3 und mehr Therapiewechsel | 3,07 | 1,45...6,51 |
| Verordnung von Reserveantibiotika insgesamt | 2,03 | 1,12...3,68 |
| Verordnung von Reserveantibiotika der $\beta$-Lactam-Gruppe | 2,36 | 1,10...5,06 |
| 5 und mehr Antibiotika verordnet | 2,22 | 1,11...4,44 |

Durch die Berechnung der Risikoverhältnisse wurde bestätigt, dass die Wahrscheinlichkeit eines Fehlschlagen der Antibiotika-Therapie bei Patienten mit einer relativ hohen ß-Lactam-Aktivität im Sputum (≥20%) signifikant um das 2-3fache höher war.
Bei einer $\beta$-Lactamase-Aktivität der Sputumproben ≥20%, wird zunächst die Verordnung von Inhibitor-geschützten ß-Lactam-Antibiotika empfohlen, die dann durch Cephalosporine der 3. Generation, Carbapenemen, Monobacten oder Kombinationen dieser ß-Lactam-Antibiotika gefolgt werden sollte. Bei unzureichender Wirkung wird der Einsatz von Antibiotika anderer pharmakologischer Gruppen (Makrolide, Glycopeptide, Oxazolidinone, Fluorchinolone, Aminoglykoside, Lincosamide etc.) empfohlen.

**Untersuchung von Speichel**

Die Untersuchung beinhaltete 77 Testproben. Das Patientenkollektiv setzte sich aus Patienten mit eitrig-entzündlichen Erkrankungen des Kiefer- und Gesichtsbereichs (n=17) und akuter eitrige Tonsillitis (n=31) zusammen. Als Probanden der Kontrollgruppe dienten gesunde Studenten.
Der Mittelwert der ß-Lactamase-Aktivität in den Speichelproben lag bei 37,2% (95% CI: 28,7...45,8) und der Median bei 21,7% (25‰ - 6,0; 75‰ - 72,0). Dabei variierte die gemessene ß-Lactamase-Aktivität von 0,0% bis zu 94,3%. Der Mittelwert der ß-Lactamase-Aktivität in der Patientengruppe mit eitrig-entzündlichen Erkrankungen des Kiefer- und Gesichtsbereichs war 45,6% (95% CI: 31,3...59,9). Für die Patientengruppe mit akuter eitriger Tonsillitis lag der Mittelwert bei 42,8% (95% CI: 29,7...55,9), während für die Kontrollgruppe ein Mittelwert von 10,4% (95% CI: 0...23,5) bestimmt wurde. Innerhalb des Patientenkollektivs wurde kein Unterschied in der Aktivität von ß-Lactamasen in den Speichelproben festgestellt (MWU-Test; p=0,77), während die ß-Lactamase-Aktivität der Kontrollen signifikant niedriger war (MWU-Test, p<0,0001).

Bakterielle Infektionen im Bereich von Kiefer- und Gesicht sowie der Mundhöhle gehen im Vergleich zu gesunden Probanden mit einer hohen ß-Lactamase-Aktivität einher. Die Produktion von ß-Lactamasen wurde bei der bakteriologischen Untersuchung bestätigt.

Eine $\beta$-Lactamase-Aktivität der Speichelproben größer als 4,0% ist ein Anzeichen einer bakteriellen Entzündung der Mundhöhle der Patienten (Sensitivität 75,0%; Spezifität 89,6%; p<0,0001). Übersteigt die $\beta$-Lactamase-Aktivität der Speichelproben 60%, ist dies ein Hinweis auf eine geringere Effizienz von ß-Lactam-Antibiotika (Sensitivität = 81,2%, Spezifität = 88,7%; p<0,0001).

**Untersuchung von Bakteriensuspensionen**

Die Untersuchung beinhaltete 131 Testproben aus Reinkulturen der folgenden Bakterienstämme: E.coli (n=45), Citrobacter freundii (n=29), Staphylococcus aureus (n=21), Proteus vulgaris (n=10), Proteus mirabilis (n=6), Salmonella enteritidis (n=5), Enterobacter cloacae (n=4), Klebsiella pneumoniae (n=4), Salmonella london (n=2), Salmonella virchov (n=2), Proteus morganii (n=1), Pseudomonas aeruginosa (n=1), Salmonella typhimurium (n=1). Antibiotikaresistenzen der isolierten Bakterienkulturen wurden mittels Diffusionsverfahren (DDV) ermittelt. Das DDV gilt als Goldstandard für den qualitativen klinischen Nachweis von Resistenzen gegenüber ß-Lactam-Antibiotika.

Es konnte für einen Großteil der Bakteriensuspensionen (n=111; 84,7%) eine ß-Lactamase-Aktivität nachgewiesen werden. Der Mittelwert der $\beta$-Lactamase-Aktivität der Bakteriensuspensionen lag bei 34,3% (95% CI: 28,0...40,5) und der Median lag bei 17,4% (25‰ - 6,6; 75‰ - 63,2).

Die Korrelationsanalyse nach Spearman zeigte einen signifikanten inversen Zusammenhang zwischen der im BIOLACTAM-Assay ermittelten $\beta$-Lactamase-Aktivität und den Inhibitionszonen der Disk-Diffusionsplatten: Ampicillin ($r_s$=-0,683; p<0,0001), mit Amoxicillin/Clavulansäure ($r_s$=-0,624; p<0,0001) und Cefotaxim ($r_s$=-0,515; p<0,0001). Mit dem Disk-Diffusionsverfahren wurde ebenfalls eine direkte Korrelation zwischen der $\beta$-Lactamase-Aktivität der Bakteriensuspensionen und einer Antibiotikaresistenz gezeigt: Ampicillin ($r_s$=0,615; p<0,0001; n=130), mit Amoxicillin / Clavulansäure ($r_s$=0,578; p<0,0001; n=130) und Cefotaxim ($r_s$=0,477; p<0,0001; n=130). Die Ergebnisse der Prüfung von Bakteriensuspensionen auf Resistenzen gegenüber ß-Lactam-Antibiotika mit Hilfe des Disk-Diffusionsverfahrens und die Ergebnisse des BIOLACTAM-Assays zeigten somit eine gute Übereinstimmung. Die beobachteten Unterschiede beruhen vermutlich auf nicht-enzymatischen Resistenzmechanismen grampositiver Mikroorganismen gegenüber ß-Lactam-Antibiotika, da der Großteil der untersuchten Mikroorganismen (n=25; 19,1%) aus grampositiven Kokken bestand.

Mit einer ROC-Analyse wurden die Ergebnisse des BIOLACTAM-Assays mit den Ergebnissen des DDV verglichen hinsichtlich Sensitivität und Spezifität verglichen (Tabelle 14). Da mittels DDV die Gesamtresistenz erfasst wird und auf die dabei ermittelten Werte bezogen wird, müssen die für den BIOLACTAM-Assay erhaltenen Werte für Sensitivität und Spezifität notwendigerweise deutlich kleiner als 100% sein.

**Tabelle 14** Zusammenfassung der Ergebnisse der ROC-Analyse und Vergleich von Sensitiviät und Spezifität des BIOLACTAM-Assav in Bezug auf das Disk-Diffusionsverfahren (=100%)

| Parameter | Cutoff-Level der $\beta$-Lactamase-Aktivität | Sensitivität im Vergleich zum Disk-Diffusionsverfahren | Spezifiität im Vergleich zum Disk-Diffusionsverfahren |
|---|---|---|---|
| Resistent gegen Penicilline und Cephalosporine 1. und 2. Generation | ≥14,2% | 76,4% (95% CI: 64,9...85,6) | 82,8% (95% CI: 70,6...91,4) |
| Resistent gegen Penicilline und Cephalosporine 1. und 2. Generation sowie Inhibitor-geschützten $\beta$-Lactam-Antibiotika | ≥26,5% | 79,2% (95% CI: 65,0...89,5) | 79,3% (95% CI: 68,9...87,4) |
| Resistent gegen Penicilline und Cephalosporine 1., 2. und 3. Generation sowie Inhibitorgeschützten $\beta$-Lactam-Antibiotika | ≥81,2% | 82,4% (95% CI: 56,6...96,0) | 94,7% (95% CI: 88,8...98,8) |

Mit dem BIOLACTAM-Assay kann untersucht werden, ob der untersuchte Bakterienstamm eine klinisch relevante Menge an ß-Lactamasen produziert, eine Resistenz gegenüber Inhibitor-geschützen ß-Lactam-Antibiotika oder Cephalosporinen der 3. Generation vorliegt oder eine Resistenz gegen ß-Lactam-Antibiotika vorliegt, die auf nicht-enzymatischen Mechanismen beruht. Dieser Ansatz erfordert die Kombination mit dem DDV.

Bei einer ß-Lactamase-Aktivität von ≥14,2% ist mit einem Wirkungsverlust von Penicillinen und Cephalosporinen der 1. und 2. Generation zu rechnen. Ab einer ß-Lactamase-Aktivität ≥26,5% liegt zudem eine Resistenz gegenüber Inhibitor-geschützen ß-Lactamen vor. Wird eine ß-Lactamase-Aktivität von 81,2% erreicht, sind auch Cephalosporine der 3. Generation unwirksam.

**Vorteile der Erfindung im Vergleich zum Stand der Technik**

Die Effizienz der quantitativen Bestimmung einer ß-Lactamase-Aktivität biologischer Flüssigkeiten und bakterieller Suspensionen zwecks Optimierung (Änderung) der antibakteriellen Therapie wird dadurch bestimmt, dass die genannte Methode einfach und nicht teuer ist. Sie verlangt keine speziellen Kenntnisse und teure Ausrüstung, zeichnet sich durch hohe Empfindlichkeit und Reproduzierbarkeit aus, liefert das Ergebnis innerhalb von 0.5-2 Stunden ab dem Moment der Probenentnahme, verlangt keine Herstellung einer Monokultur des Krankheitserregers, um dessen Resistenz gegen der ß-Lactam-Antibiotika festzustellen, ermöglicht die Erfassung der biologischen (endogenen) Resistenz (gleichgültig, ob erworben oder genetisch bedingt) gegenüber ß-Lactam-Antibiotika und ermöglicht die Untersuchung der unterschiedlichsten biologischen Flüssigkeiten (z.B. Blutserum, Sputum, Cerebrospinalflüssigkeit, Speichel, Urin, sowie von Pleura- und Aszitesflüssigkeit). Es kann die individuelle Resistenzlage des Patienten erfasst werden. Rückschlüsse sind möglich auf die Ursache der ß-Lactamase-Aktivität, ob diese genetisch bedingt ist oder erworben wurde.

Die Bestimmung der ß-Lactamase-Aktivität wird zweckmäßig unter Verwendung eines Assays durchgeführt, der aus folgenden Bestandteilen besteht: definierte Mengen an lyophilisiertem Nitrocefin, lyophilisiertem Phosphatpuffer und lyophilisierter Penicillinase, welche in destillierten Wasser gelöst werden.

Nitrocefin wird zur Verwendung in dem erfindungsgemäßen Verfahren zunächst in Dimethylsulfoxid (DMSO) gelöst. Nach Zugabe von destilliertem Wasser wird die erhaltene wässrige Lösung in Gefäße (Vials) dosiert und der lyophilen Trocknung unterzogen. Bevorzugt sind je Gefäß 125 μg Nitrocefin enthalten, die vor Gebrauch in 0,5 mL Pufferlösung aufgenommen werden ("Nitrocefin-Stammlösung"). Die Probelösung wird in den Vertiefungen der Mikrotiterplatte pipettiert und mit dem entsprechenden Anteil der verdünnten Nitrocefin-Stammlösung versetzt, im Thermostat bei 37°C 30 min lang inkubiert und die optischen Dichten (OD) anschließend mittels Plattenphotometer (Plate Reader) gemessen.

Das lyophilisierte Nitrocefin wird auch als Chromogensubstrat bezeichnet, für den Assay wird die Bezeichnung BIOLACTAM vorgeschlagen.

**Patentansprüche**

1. Verfahren zur Bestimmung von ß-Lactamase-Aktivität in biologischen Flüssigkeiten durch Selektion der Monokultur eines Krankheitserregers und dessen Identifizierung,
   Abimpfen der Monokultur in ein Nährmedium,
   Aufbringen von mit Nitrocefin imprägnierten Cellulose-Disks,
   visuelles Erfassen einer Farbänderung,
   **dadurch gekennzeichnet, dass**
   eine biologische Flüssigkeit beliebigen Ursprungs
   mit einer streng definierten Menge Nitrocefin versetzt wird
   das durch Lyphylisierung vorbereitet worden ist,
   das Gemisch bei 37°C zwischen 30 und 120 min inkubiert wird,
   ein auftretender Farbumschlag spektrophotometrisch gemessen wird,
   wodurch die Summe der ß-Lactamase-Aktivität erfasst wird
   was zu einer individuellen Therapieempfehlung führt.

2. Verfahren nach Anspruch 1, **dadurch gekennzeichnet, dass** eine Bakteriensuspension aus frischer Reinkultur eines bakteriellen Erregers hergestellt wird, 24 h bei 37°C inkubiert wird, mit einer streng definierten Menge Nitrocefin versetzt wird und der auftretende Farbumschlag spektrophotometrisch erfasst wird.

3. Verfahren nach Ansprüchen 1 und 2, **dadurch gekennzeichnet, dass** Nitrocefin in Dimethylsulfoxid gelöst und mit destilliertem Wasser verdünnt wird, die wäßrige Lösung in Gefäße, die als Vials bezeichnet werden dosiert und einer Lyophylisierung unterzogen werden, das Nitrocefin aus den Vials vor dem Gebrauch mit Pufferlösung aufgenommen wird, eine Stammlösung erzeugt und diese nach Ansprüchen 1 bis 2 benutzt wird.

4. Verfahren nach einem oder mehreren der vorhergehenden Ansprüche, **dadurch gekennzeichnet, dass** die Inkubationszeit bei Blutserum 30 min und bei Sputum, Cerebrospinalflüssigkeit, Speichel, Urin, Pleura- und Ascitesflüssigkeit 120 min beträgt.

5. Verfahren nach einem oder mehreren der vorhergehenden Ansprüche, **dadurch gekennzeichnet, dass** das spektralphotometrische Erfassen der Farbänderung bei 486 bis 505 nm erfolgt.

6. Verfahren nach einem oder mehreren der vorhergehenden Ansprüche, **dadurch gekennzeichnet, dass** die bezeichnete Bakteriensuspension beliebiger Herkunft für einen Zeitraum von 15 min bei -20°C eingefroren und 5-7 min lang in einem Thermostat bei 37°C aufgetaut wird.

7. Assay zur Verwendung bei der Bestimmung von ß-Lactamase-Aktivität, bestehend aus lyophylisiertem Nitrocefin, genau dosiert, lyophylisierter Phosphatpuffer-Lösung, lyophylisierter Penicillinase.

**Geänderte Patentansprüche gemäss Regel 137(2) EPÜ.**

1. Verfahren zur quantitativen Bestimmung von ß-Lactamase-Aktivität
   **dadurch gekennzeichnet, dass**
   in Blutserum, Sputum, Cerebrospinalflüssigkeit, Speichel, Urin, Pleura- und Ascitesflüssigkeit die Summe der exogenen und endogenen ß-Lactamase-Aktivität dergestalt bestimmt wird, dass
   eine definierte Menge biologischer Flüssigkeit mit einer streng definierten Menge Nitrocefin versetzt wird,
   das portioniert und durch Lyophylisierung vorbereitet worden ist,
   das Gemisch bei 37° C zwischen 30 min und 120 min inkubiert wird,
   ein auftretender Farbumschlag spektrofotometrisch gemessen und durch Korrelation mit einer Eichkurve quantifiziert wird
   und/oder dass zur Bestimmung der nur exogenen ß-Lactamase-Aktivität ein individualisierter Krankheitserreger in Monokultur gezüchtet wird,
   in ein Nährmedium überführt wird,
   eine definierte Menge dieser Flüssigkeit mit einer streng definierten Menge Nitrocefin versetzt wird,
   das durch Lyophylisierung vorbereitet worden ist,
   das Gemisch bei 37° C für 24 Stunden inkubiert wird,
   ein auftretender Farbumschlag spektrofotometrisch gemessen und durch Korrelation mit einer Eichkurve quantifiziert wird
   und die Analysenergebnisse miteinander korreliert werden.

2. Verfahren nach Anspruch 1, **dadurch gekennzeichnet, dass** Nitrocefin in Dimethylsulfoxid gelöst und mit destilliertem Wasser verdünnt, wird, die wässrige Lösung in Gefäße, die als Vials bezeichnet werden dosiert und einer Lyophylisierung unterzogen werden, das Nitrocefin aus den Vials vor dem Gebrauch mit Phosphatpufferlösung aufgenommen und aus der Stammlösung eine Arbeitslösung erzeugt und benutzt wird.

3. Verfahren nach einem oder mehreren der vorangehenden Ansprüche, **dadurch gekennzeichnet, dass** folgende Bakterienstämme in Reinkultur gezüchtet werden E.coli, Citrobacter freundii, Stapyhlococcus aureus, Proteus vulgaris, Proteus mirabilis, Salmonella enteritidis, Enterobacter cloacae, Klebsiella pneumoniae, Salmonella london, Salmonella virchow, Proteus morganii, Pseudomonas aeroginosa, Salmonella typhimurium,
   deren ß-Lactamase-Aktivität mittels Disk-Diffusionsverfahren DDV bestimmt wird,
   was korreliert wird mit den Ergebnissen der Summenbestimmung endogener und exogener ß-Lactamase-Aktivität,
   woraus sich eine Therapieempfehlung ergibt.

4. Verfahren nach einem oder mehreren der vorhergehenden Ansprüche, **dadurch gekennzeichnet, dass** die Inkubationszeit bei Blutserum 30 min und bei Sputum, Cerebrospinalflüssigkeit, Speichel, Urin, Pleura- und Ascitesflüssigkeit 120 min beträgt.

5. Verfahren nach einem oder mehreren der vorhergehenden Ansprüche, **dadurch gekennzeichnet, dass** das spektralfotometrische Erfassen der Farbänderung bei 486 bis 505 nm erfolgt.

6. Verfahren nach einem oder mehreren der vorangehenden Ansprüche, **dadurch gekennzeichnet, dass**
   ermittelt wird, ob in Bakteriensuspensionen eine Resistenz gegenüber inhibitorgeschützten ß-Lactam-Antibiotika oder Cephalosporinen der 3. Generation vorliegt, indem die in biologischen Flüssigkeiten als Summe der exogenen und endogenen ß-Lactamase-Aktivität bestimmten Werte
mit denen aus der Bestimmung der nur exogenen ß-Lactamase-Aktivität durch Kultivieren individualisierter Krankheitserreger in Monokultur
korreliert werden.

7. Verfahren nach einem oder mehreren der vorangehenden Ansprüche, **dadurch gekennzeichnet, dass** ermittelt wird, ob in Bakteriensuspension auf nicht enzymatischen Mechanismen beruhende Resistenz gegen ß-Lactam-Antibiotika vorliegt, indem die in biologischen Flüssigkeiten als Summe der exogenen und endogenen ß-Lactamase-Aktivität bestimmten Werte
mit denen aus der Bestimmung der nur exogenen ß-Lactamase-Aktivität durch Kultivieren individualisierter Krankheitserreger in Monokultur
korreliert werden.

8. Verfahren nach einem oder mehreren der vorhergehenden Ansprüche, **dadurch gekennzeichnet, dass** die bezeichnete Bakteriensuspension beliebiger Herkunft für einen Zeitraum von 15 min bei -20°C eingefroren und 5-7 min lang in einem Thermostat bei 37°C aufgetaut wird.

9. Assay zur Verwendung bei der Bestimmung von ß-Lactamase-Aktivität, bestehend aus portioniertem lyophylisiertem Nitrocefin, lyophylisierter Phosphatpufferlösung, lyophylisierter Katalyselösung.

10. Assay nach Anspruch 9 zur Verwendung bei der Bestimmung der ß-Lactamase-Aktivität, bestehend aus lyophylisiertem Nitrocefin in Gefäßen wie Vials/Glasfläschchen mit Bördelkappe zu je 125 µg Nitrocefin, lyophylisiertem Phosphat-Puffer in Gefäßen zu je 80,5 mg bestehend aus 13,8 mg mono-Natriumdihydrogenphosphat-Dihydrat und 66,7 mg di-Natriumhydrogen-phosphat-Dihydrat, lyophylisierter Penicillinase in Gefäßen zu je 10.000 IU sowie einer allgemein üblichen klaren Mikrotiterplatte mit 96 Vertiefungen und flachem Boden.

11. Assay nach Ansprüchen 9 und 10, **dadurch gekennzeichnet, dass** als Arbeitsmittel verwendet werden

    - 4,6 mL 0,1 M Phosphatpuffer-Lösung
    - 100 µL Penicillinase-Lösung, beide erhalten durch Zugabe von destilliertem Wasser
    - 500 µL Nitrocefin-Stammlösung nach Zugabe von 0,1 M Phosphatpufferlösung
    - 4,5 mL oder 2,5 mL Nitrocefinarbeitslösung durch Zugabe von 0,1 M Phosphatpuffer-Lösung zur Nitrocefinstammlösung.

12. Assay nach Ansprüchen 9 bis 11, **dadurch gekennzeichnet, dass** mittels Plattenphotometer die optische Dichte einer inkubierten Probe gemessen wird.

13. Assay nach Ansprüchen 9 bis 12, **dadurch gekennzeichnet, dass** eine durch hydrolytische Spaltung des ß-Lactam-Ringes von Nitrocefin, die einer ß-Lactamase-Aktivität ≥ 70 % entspricht, in einer Blutserumprobe einen Therapiewechsel indiziert.

14. Assay nach Ansprüchen 9 bis 12, **dadurch gekennzeichnet, dass** eine hydrolytische Spaltung des ß-Lactam-Ringes von Nitrocefin, die einer ß-Lactamase-Aktivität ≥ 20 % entspricht, in Sputum und Cerebrospinalflüssigkeit einen Therapiewechsel indiziert.

Europäisches
Patentamt

European
Patent Office

Office européen
des brevets

**EUROPÄISCHER RECHERCHENBERICHT**

**Nummer der Anmeldung**

EP 13 00 4963

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | C CONNELL ET AL: "Detection of beta lactamase in sputum.", JOURNAL OF CLINICAL PATHOLOGY, Bd. 47, Nr. 8, 1. August 1994 (1994-08-01), Seiten 732-735, XP055092987, ISSN: 0021-9746, DOI: 10.1136/jcp.47.8.732 | 1,2,4 | INV. C12Q1/18 C12Q1/34 |
| Y | * Zusammenfassung * * Seite 732, Spalte 2, Absatz 2 - Seite 733, Spalte 1, Absatz 2 * ----- | 3,5-7 | |
| X | P. DRAGICEVIC ET AL: "Activities and sources of beta-lactamase insputum from patients with bronchiectasis.", JOURNAL OF CLINICAL MICROBIOLOGY, Bd. 27, Nr. 5, 1. Mai 1989 (1989-05-01), Seiten 1055-1061, XP055093011, | 1,2,4,5 | |
| Y | * Zusammenfassung * * Materials and Methods section * ----- | 3,6,7 | |
| Y | W H BOUGHTON: "Rapid detection in spinal fluid of beta-lactamase produced by ampicillin-resistant Haemophilus influenzae", J. CLIN. MICROBIOL, Bd. 15, Nr. 6, 1. Januar 1982 (1982-01-01), Seiten 1167-1168, XP055093027, * das ganze Dokument * ----- | 1-7 | **RECHERCHIERTE SACHGEBIETE (IPC)** C12Q |

-/--

Der vorliegende Recherchenbericht wurde für alle Patentansprüche erstellt

| Recherchenort | Abschlußdatum der Recherche | Prüfer |
|---|---|---|
| München | 12. Dezember 2013 | Thumb, Werner |

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 13 00 4963

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | Semenov et al.: "Biological resistance to beta-lactam antibiotics, methods of registration", , 12. Juni 2013 (2013-06-12), XP002717840, Gefunden im Internet: URL:http://sivital.by/en/articles_en/biological-resistance-to-beta-lactam-antibiotics-methods-of-registration.html [gefunden am 2013-12-06] * das ganze Dokument * | 1-7 | |
| Y | SEMENOV V M ET AL: "The clinical significance of biologic resistance to lactam antibiotics", MEDITSENSKKI ZHURNAL UZBEKISTANA, UZ, Nr. 1, 1. Januar 2012 (2012-01-01), Seiten 84-90, XP009174875, ISSN: 0025-830X * Zusammenfassung * | 1-7 | |
| A | D. M. LIVERMORE ET AL: "Detection of -lactamase-mediated resistance", JOURNAL OF ANTIMICROBIAL CHEMOTHERAPY, Bd. 48, Nr. suppl 1, 1. Juli 2001 (2001-07-01), Seiten 59-64, XP055093033, ISSN: 0305-7453, DOI: 10.1093/jac/48.suppl_1.59 * Zusammenfassung * * Seite 60, Spalte 1, Absatz 2-3 * | 1-7 | RECHERCHIERTE SACHGEBIETE (IPC) |
| A | US 2009/117601 A1 (YANG-WOYTOWITZ MEI [US] ET AL) 7. Mai 2009 (2009-05-07) * das ganze Dokument * | 1-7 | |

Der vorliegende Recherchenbericht wurde für alle Patentansprüche erstellt

| Recherchenort | Abschlußdatum der Recherche | Prüfer |
|---|---|---|
| München | 12. Dezember 2013 | Thumb, Werner |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 13 00 4963

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-12-2013

| Im Recherchenbericht angeführtes Patentdokument | Datum der Veröffentlichung | Mitglied(er) der Patentfamilie | | Datum der Veröffentlichung |
|---|---|---|---|---|
| US 2009117601 A1 | 07-05-2009 | AT | 539165 T | 15-01-2012 |
| | | EP | 2207893 A1 | 21-07-2010 |
| | | JP | 2011500057 A | 06-01-2011 |
| | | US | 2009117601 A1 | 07-05-2009 |
| | | US | 2012077215 A1 | 29-03-2012 |
| | | WO | 2009051838 A1 | 23-04-2009 |

EPO FORM P0461

Für nähere Einzelheiten zu diesem Anhang : siehe Amtsblatt des Europäischen Patentamts, Nr.12/82